# EUROPÄISCHE PATENTSCHRIFT

(11) **EP 2 745 937 B1**
(45) Veröffentlichungstag und Bekanntmachung des Hinweises auf die Patenterteilung: **04.05.2016**
(21) Anmeldenummer: 13005845.6
(22) Anmeldetag: 16.12.2013
(51) Int. Cl.: B01L 3/00, A61B 10/00, G01N 33/00

(54) **Probenbehälter**
Sample container
Récipient à échantillon

(30) Priorität: 21.12.2012 CH 29262012
(43) Veröffentlichungstag der Anmeldung: 25.06.2014
(73) Patentinhaber: Berlinger & Co., 9608 Ganterschwil (CH)
(72) Erfinder: Oertli, Christian, CH-9621 Oberhelfenschwil (CH)
(74) Vertreter: Quehl, Horst Max

(56) Entgegenhaltungen:
- WO-A1-2010/075814
- WO-A2-2011/071364
- US-A- 3 579 306
- US-A- 4 180 009
- US-A- 4 409 182
- US-A1- 2007 259 442
- US-A1- 2008 105 066

## Beschreibung

Die Erfindung betrifft einen Probenbehälter für eine zu testende Flüssigkeit mit den Merkmalen des Oberbegriffs des Patentanspruchs.

Ein durch die WO 2011/071364 bekannter Probenbehälter dieser Art hat einen in den Probenbehälter eingesetzten und diesen nahezu vollständig ausfüllenden, becherförmigen Anzeigeträger, dessen speziell für ihn geformten Anzeigemittel sich grossflächig über dessen gesamte Höhe erstrecken und die nach unten in einer Reihe von zueinander parallelen, streifenförmigen Fortsätzen enden. Ein zentral vom Boden dieses Anzeigeträgers nach oben ragender Dorn soll den Boden eines zusätzliche von oben eingesetzten Probenbechers perforieren, so dass sich ein Zufluss der zu testenden Flüssigkeit zu den Anzeigemitteln des becherförmigen Anzeigeträgers ergibt.

Durch die US 2007/0259442 und die US 2008/0105066 sind Probenbehälter bekannt deren Anzeigemittel aus zahlreichen Teststreifen bestehen, die durch zusätzliche Streifenhalter parallel zueinander entlang von Mantellinien des Probenbehälters in diesem befestigt sind.

Ausserdem ist es bekannt für die Aufnahme der zu testenden Flüssigkeit einen einfach gestalteten Becher als Probenbehälter zu verwenden und als Anzeigemittel einen eine Anzahl von reaktiven Testfeldern aufweisenden Teststreifen in diesen einzutauchen. Ein solcher Teststreifen wurde somit bisher separat zur Verfügung gestellt. Auftretende Farbänderungen von am Teststreifen vorgesehenen Testfelder ergeben dann einen Hinweis auf bestimmte, in der Flüssigkeit enthaltene Substanzen.

Der Erfindung liegt die Aufgabe zugrunde einen Probenbehälter der genannten Art zu finden, der für einen Schnelltest, bei einfacher Art seiner Handhabung, auch die Verwendung einer relativ geringen Menge zu testender Flüssigkeit ermöglich, der mit verhältnismässig geringem Aufwand herstellbar ist und der eine grosse Fälschungssicherheit gewährleistet.

Die Lösung der Aufgabe der Erfindung erfolgt aufgrund der Merkmalen des Patentanspruchs 1. Vorteilhafte Ausführungsformen der Erfindung sind Gegenstand der abhängigen Patentansprüche und der folgenden Beschreibung anhand eines in den Zeichnungen dargestellten Ausführungsbeispieles zu entnehmen.

Es zeigt:

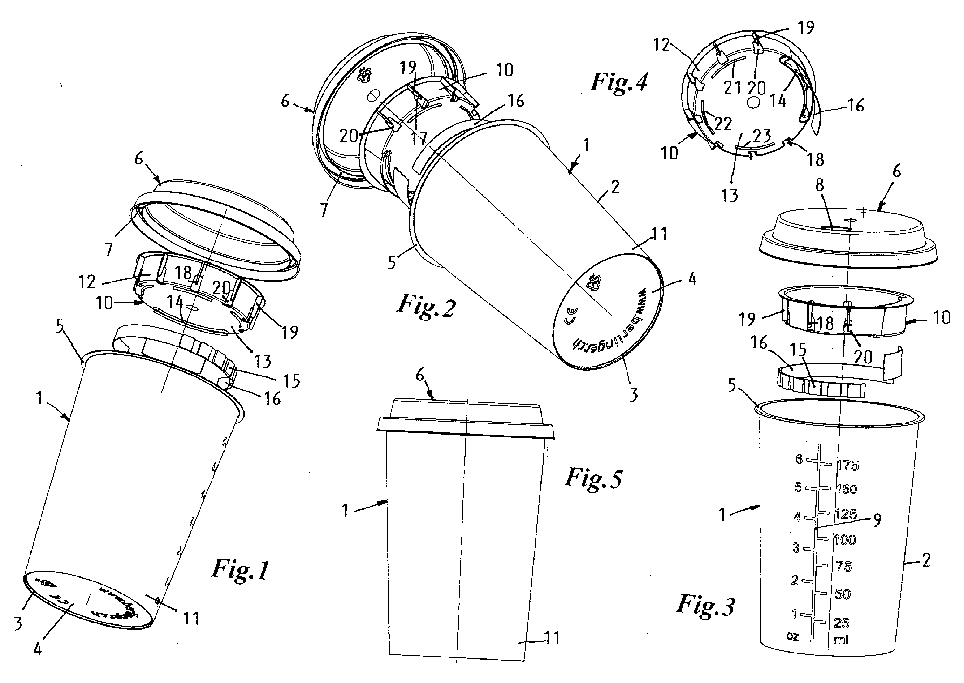

Fig.1 eine geneigte perspektivische Darstellung der Einzelteile eines erfindungsgemässen Probenbehälters mit achsgleicher gegenseitiger Zuordnung,
Fig.2 eine Darstellung entsprechend Fig.1 unter einem anderem Blickwinkel,
Fig.3 eine Darstellung entsprechend Fig. 1 und Fig.2 bei vertikaler Ausrichtung,
Fig.4 eine perspektivische Darstellung eines Anzeigeträgers des Probenbehälters der Fig. 1 bis 3 und
Fig.5 eine Seitenansicht des geschlossenen Probenbehälters nach Fig. 1 bis 3.

Der Probenbehälter 1 des dargestellten Ausführungsbeispieles hat becherförmig eine konische Umfangswand 2, eine ebene, über einen Bodenrand 3 gering nach innenversetzte Bodenwand 4 und einen flanschförmig nach aussen abstehenden Öffnungsrand 5.

Zum Verschliessen des Probenbehälters 1 ist eine Verschlusskappe 6 mit einer umlaufenden Innenrille 7 vorgesehen, die über den Öffnungsrand 5 des Probenbehälters einrastbar ist. Ausserdem hat die Verschlusskappe 6 in Randnähe eine Ausgussöffnung 8, die nach Fertigstellung eines Schnelltestes im Probenbehälter das Umgiessen in einen mit einem Garantieverschluss versehenen Behälter für die Archivierung oder für weitere Tests erleichtert.

Der Probenbehälter 1 ist vorzugsweise gesamthaft im Tiefziehverfahren aus einem durchsichtigen Kunststoffmaterial geformt, so dass der Füllstand einer eingefüllten, zu testenden Flüssigkeit an einer an der Behälterwand 2 vorgesehenen Messskala 9 ablesbar ist.

Um zusätzlich zur Füllstandsmessung auch eine Messung weiterer Eigenschaften einer eingefüllten Flüssigkeit innerhalb des Probenbehälters 1 zu ermöglichen, ist in diesem ein Anzeigemittel aufweisender Anzeigeträger 10 eingeschlossen. Dieser hat napfförmig eine dem unteren Bereich 11 des Probenbehälters 1 angepasste Umfangswand 12 und einen zum Kontakt und Schweissverbindung mit der Bodenwand 4 des Probenbehälters 1 bestimmten Napfboden 13. Dabei dient die somit an der Innenfläche der konischen Umfangswand 2 des Probenbehälters 1 anliegende und von aussen sichtbare Umfangswand 12 des Anzeigeträgers 10 der Aufnahme und Befestigung der weiteren Anzeigemittel.

Im dargestellten Ausführungsbeispiel der Erfindung ist durch einen Doppelwandbereich der Umfangswand 12 des Anzeigeträgers 10 taschenförmig eine Aufnahme 14 für einen skalenartigen Temperaturmesstreifen vorgesehen, so dass die Temperatur der in den Probenbehälter 1 eingefüllten, zu testenden Flüssigkeit durch die durchsichtigen Umfangswände 12, 2 des Anzeigeträgers 10 sowie des Probenbehälters hindurch vom Temperaturmessstreifen ablesbar ist.

Ausserdem dient die Umfangswand 12 des Anzeigeträgers der Aufnahme und Befestigung eines quadratförmige Testfelder 15 aufweisenden Teststreifens 16 für weitere Eigenschaften der Flüssigkeit, wie sie z.B. für einen Dopingschnelltest aus Urin ermittelbar sind.

Um einen handelsüblichen Teststreifen 16 auf einfache Weise an der Umfangswand 12 des Anzeigeträgers 10 befestigen zu können, sind entlang dieser Umfangswand 12 mit gleichem Abstand voneinander mehrere Streifenhalter 17 angeformt, so dass z.B. zwischen ihnen jeweils zwei Testfelder 15 eines unter die Streifenhalter 17 eingeschobenen Teststreifens 16 angeordnet sind. Dabei bilden die einzelnen Streifenhalter 17 ein freies Ende 18 von aussen an der Umfangswand 12 des Anzeigeträgers 10 angeformten, achsparallelen Rippen 19.

Die Ausformung der freien Enden 18 dieser Streifenhalter 17 wird dadurch vereinfacht, dass der Anzeigeträger 10 in ihrem Bereich Wandöffnungen 20 aufweist.

Nach Aufschweissen des Napfbodens 13 eines auf solche Weise ausgerüsteten Anzeigeträgers 10, z.B. entlang von bogenförmigen Schweisskontaktlinien 21, 22,23 ist der in der taschenförmigen Aufnahme 14 eingeschlossene Temperaturmesstreifen und der Testfelder 15 aufweisende Teststreifen 16 sicher bzw. unzugänglich im Probenbehälter 1 integriert und durch die Behälterwand 2 hindurch ablesbar.

## Patentansprüche

1. Probenbehälter für eine zu testende Flüssigkeit, mit Anzeigemitteln (16) für mindestens eine Eigenschaft der Flüssigkeit, wobei in dessen Innenraum mindestens ein Anzeigemittel (16) aufweisender Anzeigeträger (10) eingeschlossen und befestigt ist und der Probenbehälter (1) zumindest in einem Wandbereich aus einem durchsichtigen Material geformt ist, so dass das mindestens eine Anzeigemittel (16) durch diesen Wandbereich hindurch sichtbar ist, **dadurch gekennzeichnet, dass** der Anzeigeträger (10) flach napfförmig ausgebildet ist und mit der Bodenwand (4) des Probenbehälters (1) verschweisst ist und als Anzeigemittel ein Teststreifen (16) mit Testfeldern (15) vorgesehen ist und dieser unter mehreren, an der Umfangswand (12) des Anzeigeträgers (10) als Rippen (19) angeformten und frei endenden Streifenhaltern (17) eingesetzt ist.

2. Probenbehälter nach Anspruch 1, **dadurch gekennzeichnet, dass** an der Umfangswand (12) des Anzeigeträgers (10) eine zu ihr gleichlaufende, taschenförmige Aufnahme (14) für ein streifenförmiges Anzeigemittel angeformt ist.

3. Probenbehälter nach Anspruch 1 bis 2, **dadurch gekennzeichnet, dass** er einen flanschförmig nach aussen abstehenden Öffnungsrand (5) aufweist und dieser in einer Innenrille (7) einer Verschlusskappe (6) verrastet ist.

4. Probenbehälter nach Anspruch 3, **dadurch gekennzeichnet, dass** die Verschlusskappe (6) in Randnähe eine Ausgussöffnung (8) aufweist.

5. Probenbehälter nach Anspruch 1, **dadurch gekennzeichnet, dass** in der Umfangswand (12) des Anzeigeträgers (10) im Bereich der Streifenhalter (17) Öffnungen (20) vorgesehen sind.

## Claims

1. Sample container for a liquid to be tested, having display means (16) for at least one property of the liquid, wherein at least one display carrier (10) having display means (16) is included and fastened in the inner space thereof and the sample container (1) is formed from a transparent material at least in one wall region, such that the at least one display means (16) is visible through this wall region, **characterised in that** the display carrier (10) is formed to be flat and bowl-shaped and is welded to the base wall (4) of the sample container (1) and a test strip (16) with test fields (15) is provided as a display means and this is used among several strip holders (17) which are formed on the peripheral wall (12) of the display carrier (10) as ribs (19) and end freely.

2. Sample container according to claim 1, **characterised in that** a bag-like receiver (14) for a strip-shaped display means is formed on the peripheral wall (12) of the display carrier (10), said bag-like receiver (14) running parallel to the peripheral wall (12).

3. Sample container according to claim 1 to 2, **characterised in that** it has an opening edge (5) which projects outwards in a flange shape and is locked in place in an inner groove (7) of a sealing cap (6).

4. Sample container according to claim 3, **characterised in that** the sealing cap (6) has a spout opening (8) in the vicinity of the edge.

5. Sample container according to claim 1, **characterised in that** openings (20) are provided in the peripheral wall (12) of the display carrier (10) in the region of the strip holders (17).

## Revendications

1. Réservoir à essai destiné à recevoir un liquide à tester, doté d'un moyen d'affichage (16) pour au moins une propriété du liquide, dans l'espace intérieur duquel un support d'affichage (10) avec au moins un moyen d'affichage (16) est inclus et fixé, et le réservoir à essai (1) est formé au moins sur une paroi d'un matériau transparent, de sorte qu'au moins un moyen d'affichage (16) est visible à travers cette paroi, **caractérisé en ce que** le support d'affichage (10) est plat en forme de coupelle et est soudé avec le fond (4) du réservoir à essai (1), et **en ce qu'**une bande de test (16) avec des zones de test (15) est prévue comme moyen d'affichage et **en ce que** celle-ci, parmi d'autres, est formée comme une nervure (19) au niveau de la paroi périphérique (12) du support d'affichage (10) et est disposée au niveau de plusieurs supports de bandes (17) à extrémités libres.

2. Réservoir à essai selon la revendication 1, **caractérisé en ce qu'**au niveau de la paroi périphérique (12) du support d'affichage (10), un logement (14) en forme de poche parallèle à la paroi et destiné à un moyen d'affichage en forme de bande est formé.

3. Réservoir à essai selon l'une quelconque des revendications 1 à 2, **caractérisé en ce qu'**il comporte un bord d'ouverture (5) orienté vers l'extérieur en forme de collerette et **en ce que** celui-ci est enclenché dans une rainure (7) d'un capuchon (6).

4. Réservoir à essai selon la revendication 3, **caractérisé en ce que** le capuchon (6) comporte une ouverture en forme de bec (8) à proximité du bord.

5. Réservoir à essai selon la revendication 1, **caractérisé en ce que** des ouvertures (20) sont prévues dans la paroi périphérique (12) du support d'affichage (10), dans la région du support de bandes (17).
